Europäisches Patentamt

European Patent Office

Office européen des brevets

(11) Veröffentlichungsnummer: **0 516 963 A2**

## (12) EUROPÄISCHE PATENTANMELDUNG

(21) Anmeldenummer: **92106999.3**

(22) Anmeldetag: **23.04.92**

(51) Int. Cl.5: **A61L 2/20**

(30) Priorität: **27.05.91 DE 4117306**

(43) Veröffentlichungstag der Anmeldung:
**09.12.92 Patentblatt 92/50**

(84) Benannte Vertragsstaaten:
**AT BE CH DE ES FR GB IT LI NL**

(71) Anmelder: **AIR PRODUCTS GMBH**
**Hüttenstrasse 50**
**W-4320 Hattingen(DE)**
Anmelder: **HERCO KÜHLTECHNIK HERMANNS**
**& CO. GmbH**
**Rudolf-Diesel-Strasse 28**
**W-4230 Wesel(DE)**

(72) Erfinder: **Karthaus, Michael, Dipl.-Ing.**
**Im Winkel 12**
**W-4040 Neuss(DE)**
Erfinder: **Babel, Olaf, Dipl.-Ing.**
**Freiheit 5**
**W-4320 Hattingen(DE)**
Erfinder: **Hermanns, Peter, Dr. rer.nat.**
**Teichstege 10**
**W-4230 Wesel(DE)**
Erfinder: **Hermanns, Klaus, Dr. rer.nat**
**Schwarzensteiner Weg**
**W-4224 Hünxe 2(DE)**
Erfinder: **Kusenberg, Gerhard, Ing.**
**Am Kiefernhügel 6**
**W-4230 Wesel(DE)**
Erfinder: **Hagenbruck, Norbert, Dipl.-Ing.**
**Wannerstrasse 19**
**W-4200 Oberhausen 11(DE)**

(74) Vertreter: **Schwabe - Sandmair - Marx**
**Stuntzstrasse 16**
**W-8000 München 80(DE)**

(54) **Verfahren und Anlage zur Rückgewinnung eines Sterilisiergases.**

(57) Bei einem Verfahren und einer Anlage zur Rückgewinnung eines Sterilisiergases, insbesondere von Ethylenoxid, werden zu sterilisierende Materialien in einer Sterilisierkammer mit dem Sterilisiergas sterilisiert; das aus der Sterilisierkammer abgesaugte Sterilisiergas/Luft-Gemisch wird in einem Vorkühler vorgetrocknet; das vorgetrocknete Sterilisiergas/Luft-Gemisch wird in einem Trockner von Restfeuchtigkeit befreit, das Sterilisiergas in einem Tieftemperaturkondensator verflüssigt und bis zur Wiederverwendung in der Sterilisierkammer in einem Lagerbehälter gespeichert. Dabei läuft das Sterilisieren und die gesamte Rückgewinnung des Sterilisiergases bei Drücken unterhalb des die Anlage umgebenden Atmosphärendruckes ab.

Fig. 1

Die Erfindung betrifft ein Verfahren und eine Anlage zur Rückgewinnung eines Sterilisiergases, insbesondere Ethylenoxid.

Zur Sterilisation von medizinischen Geräten, auch verpackten, ist es üblich, die zu sterilisierenden Materialien in einer Sterilisierkammer einem Sterilisiergas, üblicherweise ein Alkylenoxid, auszusetzen. Das meistens verwendete Ethylenoxid ist ein sehr toxisches und in Verbindung mit Luft leicht entflammbares und explosibles Gas mit einer unteren Explosionsgrenze von 2,6 % und einer oberen Explosionsgrenze von 100 % Ethylenoxidgehalt in einem Ethylenoxid/Luft-Gemisch. Auch ohne Luftzusatz kann daher reines Ethylenoxid (ETO) im Beisein einer geeigneten Zündquelle explodieren. Um die damit verbundenen Risiken zu begrenzen, hat es sich durchgesetzt, ETO mit einem relativ inerten Gas zu verdünnen. Typisch ist die Mischung von 12 Gew.-% ETO mit 88 Gew.-% Freon R 12 (Dichlordifluormethan).

Um die toxisch wirkenden und relativ teuren Bestandteile eines solchen Gemisches nicht nach jedem Sterilisierzyklus in die Umgebung abgeben zu müssen, wurden in den vergangenen Jahren Verfahren und Anlagen zur Rückgewinnung des Sterilisiergases und der erwähnten inerten Zusatzgase entwickelt. So gehen aus der US-PS 3 549 312 ein Verfahren sowie eine Anlage zur Rückgewinnung von Alkylenoxid und den beigemischten inerten Bestandteilen hervor, bei denen das aus einer Sterilisierkammer abgesaugte Gemisch von Alkylenoxid und den inerten Beimischungen einem Vorkühler und anschließend einem Absorber zugeführt werden. Das in dem Absorber von Feuchtigkeit befreite Gemisch gelangt dann in einen Kondensator zur Verflüssigung des Alkylenoxids und des beigemischten Freon R 12 und schließlich in ein Reservoir zur Zwischenspeicherung des flüssigen Gemisches. Aus dem unter Überdruck stehenden Reservoir kann dieses Gemisch schließlich wieder der Sterilisierkammer zugeführt werden. Obwohl in dieser Druckschrift durchaus vom Gebrauch reinen Ethylenoxids als Sterilisiergas die Rede ist, dürfte ein nach dieser Lehre arbeitendes Verfahren oder eine Anlage auf die Verwendung eines Gemisches aus Ethylenoxid und einem relativ inerten Gas beschränkt bleiben, da keine Maßnahmen erkennbar sind, der Problematik der leichten Entflammbarkeit von reinem Ethylenoxid zu begegnen und Überdruck in der Sterilisierkammer und der nachgeschalteten Rückgewinnung zu vermeiden. Dies jedoch kann zum Austritt der toxischen Gase in die Umgebung führen.

Weitere Verfahren und Anlagen zur Rückgewinnung eines Sterilisergases oder eines Gemisches aus einem Sterilisiergas und zumeist einem gasförmigen, fluorierten Kohlenwasserstoff sind durch die EP-A-0 130 319, die US-PS 3 989 461 und die EP-A-0 326 985 bekannt. Alle dort offenbarten Verfahren bzw. Anlagen arbeiten zumindest zeitweise und in Teilbereichen solcher Anlagen bei Drücken oberhalb des Umgebungsdrucks, wodurch ständig Gefahr von Leckverlusten gegeben ist. Die Möglichkeit, reines Ethylenoxid als Sterilisiergas zu verwenden, wird zwar auch in der EP-A0 130 319 angeschnitten, die besondere Problematik bei dessen Handhabung und insbesondere deren technische Lösung werden jedoch nicht erwähnt. Offenbar sind sie nicht erkannt worden.

Es ist daher die Aufgabe der vorliegenden Erfindung, die Nachteile der aus dem Stand der Technik bekannten Verfahren und Anlagen zur Rückgewinnung von Sterilisiergasen zu vermeiden. Insbesondere sollen solch ein Verfahren bzw. eine Anlage den sicheren Betrieb mit reinem Ethylenoxid als Sterilisiergas ermöglichen.

Diese Aufgabe wird durch den Gegenstand des kennzeichnenden Teils des Verfahrensanspruchs 1 sowie des Vorrichtungsanspruchs 11 gelöst.

Durch den Verzicht auf die üblicherweise einem Sterilisiergas beigemischten Fluorchlorkohlenwasserstoffe, wie beispielsweise Freon, wird zunächst ein Beitrag zur Reduzierung der Schädigung der Ozonschicht durch die in die Umgebung entweichenden Fluorchlorkohlenwasserstoffe geleistet.

Die Verwendung eines reinen Sterilisiergases, insbesondere von reinem Ethylenoxid (ETO), ermöglicht eine starke Herabsetzung der Verweildauer der zu sterilisierenden Materialien in einer Sterilisierkammer, da die Konzentration des Sterilisiergases im Sterilisiergas-Luft-Gemisch der Sterilisierkammer nun natürlich höher als bei Verwendung der vorgenannten Beimischungen ist.

Ferner kann eine unter Unterdruck betriebene Sterilisierkammer rascher evakuiert werden als die aus dem Stand der Technik bekannten und bei überdruck betriebenen Sterilisierkammern, wodurch weiterhin die Verweildauer der zu sterilisierenden Materialien in solch einer Kammer vermindert und die Auslastung der Sterilisierkammer erhöht werden können. Da das gesamte Verfahren bzw. die gesamte Anlage zur Rückgewinnung des Sterilisiergases bei Drücken arbeitet, die unterhalb des Umgebungsdrucks liegen, wird die Betriebssicherheit hinsichtlich der Gefahr des Entweichens von Sterilisiergas erhöht. Etwaige Leckagen innerhalb einer erfindungsgemäßen Anlage führen somit nur zum Eintritt von Luft, aber nicht zum ETO-Austritt in die Atmosphäre.

Gerade die Verwendung von reinem ETO als Sterilisiergas wirft aufgrund seiner leichten Entflammbarkeit und Explosibilität besondere Probleme auf. Um das als Sterilisiergas bevorzugte ETO verwenden zu können (und im folgenden soll stellvertretend nur noch von ETO die Rede sein), wird das aus der Sterilisierkammer abgezogene ETO/Luft-Gemisch über einen Vorkühler einem Trockner zugeführt und dort durch Adsorption in einem kühlbaren Molekularsieb weiter von noch im Gemisch vorhandener Restfeuchtigkeit befreit. Die Adsorption von Wasserdampf im Molsieb ist exotherm. Ferner wird etwas ETO ebenfalls exotherm koadsorbiert. Diese Koadsorption nimmt mit steigender Temperatur rasch zu, so daß die Temperatur bei Verwendung von Rein-ETO beschleunigt bis zur Explosionsgrenze ansteigen kann. Bei der Verwendung des üblichen Gemisches aus 12 % ETO und 88 % Freon R 12 verhindert die Kühlwirkung des Freons einen Anstieg der Temperatur in solch einem Molekularsieb. Bei reinem ETO muß nun dagegen mit einem gefährlichen Temperaturanstieg, bis hin zur Explosionsgrenze des reinen ETO, gerechnet werden. Während bei einer eventuellen Explosion eine Fortpflanzung in Strömungsrichtung, d.h. in Richtung auf den der Verflüssigung des ETO dienenden Teils der Anlage, wegen der dort herrschenden sehr tiefen Temperaturen ausgeschlossen werden kann, muß die Fortpflanzung einer eventuellen Explosion zurück in Richtung zur Sterilisierkammer ("Rückschlag") ernsthaft in Betracht gezogen werden. Zur Verhinderung dieses Rückschlages wird das Molekularsieb erfindungsgemäß während des Betriebs, also in der Adsorptions - phase, gekühlt, um einen unzulässigen Temperaturanstieg im Molekularsieb von vornherein zu unterbinden und die Koadsorption von ETO auf den geringstmöglichen Wert zu begrenzen.

Ferner ist in einer besonders vorteilhaften Ausgestaltung der Erfindung eine wirksame Sperre gegen die Fortpflanzung einer etwaigen Explosion und der damit verbundenen Zerfallsreaktionen von ETO vom Molekularsieb in Richtung zur Sterilisierkammer vorgesehen. Bevorzugt stellt ein zwischen Sterilisierkammer und Molekularsieb angeordneter Vorkühler solch eine Sperre dar. Erfindungsgemäß werden zu diesem Zweck die das ETO/Luft-Gemisch führenden Rohre innerhalb des Vorkühlers durch gegenseitige Abstimmung von Strömungsquerschnitt und Strömungslänge entsprechend dimensioniert.

Die Erfindung wird nachstehend anhand einer bevorzugten Ausführungsform unter Bezugnahme auf die Zeichnungen im einzelnen erläutert. Dabei werden weitere Vorteile und Merkmale der vorliegenden Erfindung offenbart. Es zeigen:

Fig. 1    eine schematische Darstellung einer Rückgewinnungsanlage;

Fig. 2    eine Darstellung eines herkömmlichen Molekularsiebes im Längsschnitt;

Fig. 3    eine Darstellung eines gekühlten Molekularsiebes im Längsschnitt; und

Fig. 4    das Molekularsieb nach Fig. 3 im Querschnitt.

Zu Beginn eines Sterilisierzyklus wird über ein absperrbares Ventil 5 feuchte Umgebungsluft in eine Sterilisierkammer 10 eingelassen, um zuerst das Wachstum von im nachfolgenden Sterilisierprozeß abzutötenden Keimen zu ermöglichen. Nachdem auf diese Art ein für die Sterilisierung notwendiger definierter Ausgangszustand in der Sterilisierkammer 10 erreicht ist, wird die Sterilisierkammer 10 zunächst durch eine Pumpe 15 auf ca 100 mbar evakuiert, bevor schließlich über den Anwärmer bzw. Verdampfer 90 solange Sterilisiergas in die Sterilisierkammer 10 geleitet wird, bis dort ein Absolutdruck von ca. 800 mbar erreicht ist. Der Kammerdruck bleibt somit deutlich unter dem Druck der umgebenden Atmosphäre.

Im Anschluß an die Sterilisierung wird die Sterilisierkammer 10 durch eine Vakuumpumpe 75 abgesaugt. Das abgesaugte Sterilisiergas/Luft-Gemisch, im folgenden soll stellvertretend für Sterilisiergas nur noch von Ethylenoxid (ETO) die Rede sein, passiert zunächst einen Vorkühler 20, in dem es auf ca. 4° bis 10° C abgekühlt wird. Im Vorkünler 20 wird der überwiegende Teil des im ETO/Luft-Gemisch enthaltenden Wasserdampfes verflüssigt und in einem nachfolgenden Wasserabscheider 25 abgeschieden. Die Fremdkälte zur Vorkühlung wird durch einen Verdampfer 30 einer Kältemaschine über einen Wasser- oder Sole-Kreislauf zur Verfügung gestellt. Das ETO/Luft-Gemisch strömt nun zu einem Trockner 40, der in der dargestellten Ausführungsform als Feuchtigkeit adsorbierendes Molekularsieb 41 bzw. 42 ausgebildet ist. Dabei weist solch ein Trockner 40 zwei abwechselnd betreibbare Molekularsiebe 41 und 42 auf. Die Adsorptionsphase des einen Molekularsiebs wird als Regenerationsphase des jeweils anderen Molekularsiebs genutzt. Im gerade adsorbierenden Molekularsieb 41 oder 42 wird die noch im bereits vorgetrockneten ETO/Luft-Gemisch enthaltende Feuchtigkeit soweit adsorbiert, daß der Taupunkt des den Trockner 40 verlassenden ETO/Luft-Gemisches im Bereich zwischen -80° C und -100° C liegt. Das jeweils aktive Molekularsieb 41 oder 42 wird dabei wie der Vorkühler 20 ebenfalls durch den Verdampfer 30 der Kältemaschine gekühlt.

Vom Trockner 40 gelangt das weitestgehend von Feuchtigkeit befreite ETO/Luft-Gemisch nunmehr in einen mit flüssigem Stickstoff oder einem anderen geeigneten Kältemittel betriebenen Tieftemperaturkondensator 50, in dem das ETO/Luft-Gemisch zur Verflüssigung von ETO auf eine Temperatur im Bereich von -80° C und -130° C abgesenkt wird. Das bis auf geringste Spuren verflüssigte ETO gelangt vom Tieftemperaturkondensator 50 entweder direkt oder über einen dem Tieftemperaturkondensator 50 nachgeschalteten Kondensatabscheider 60 in eine ETO-Vorlage 78. Von dort wird das verflüssigte ETO durch eine Pumpe 81 in den Lagerbehälter 80 abgepumpt. Während des Sterilisierens und der Rückgewinnung steht das gesamte System außer dem Lagerbehälter 80 zwischen den Motorventilen 8a und 84 unter Unterdruck.

Die nicht kondensierbaren Bestandteile des ETO-Luft/Gemisches, im wesentlichen Luft, werden aus dem Tieftemperaturkondensator 50 über einen Anwärmer 70 von der Vakuumpumpe 75 abgezogen und in die Umgebung abgegeben. Im nächstfolgenden Sterilierzyklus gelangt dann das flüssige ETO aus dem Lagerbehälter 80 über eine Leitung 82 und das absperrbare Ventil 84 zu dem ETO-Verdampfer 90 und von dort schließlich gasförmig zur Sterilisierkammer 10.

Wie sich bei Probeläufen einer solchen Anlage zeigte, kommt es beim Betrieb mit reinem ETO als Sterilisiergas zu unzulässig hohen Temperaturen in den Molekularsieben 41 und 42. Der Überhitzung der Molekularsiebe 41 bzw. 42 wird durch aktive Kühlung des Adsorptionsbereichs eines solchen Siebs vorgebeugt.

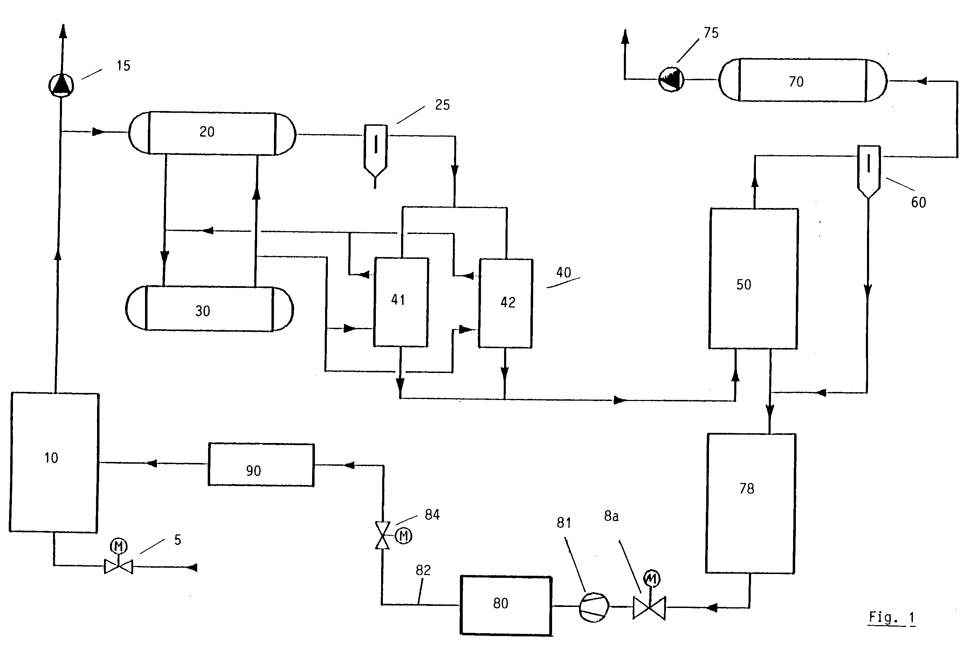

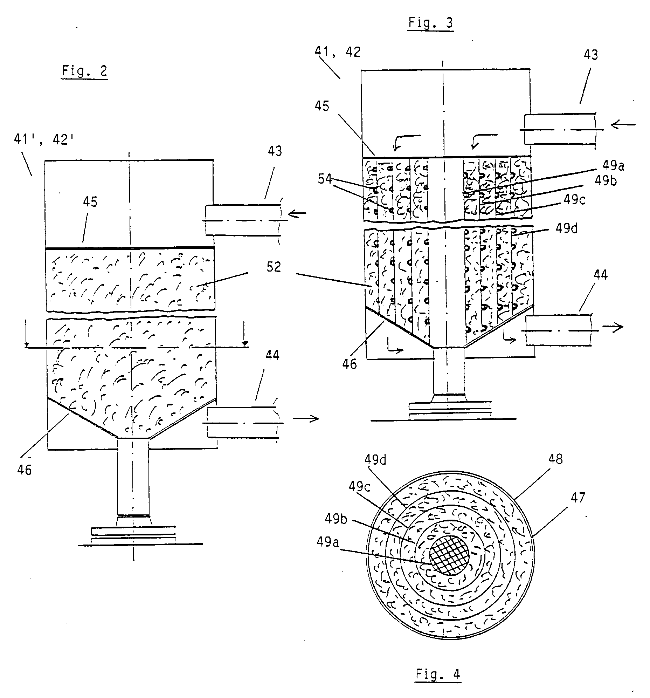

In Figur 2 ist ein herkömmliches Molekularsieb 41' bzw. 42' im Schnitt dargestellt. Diese Konstruktion reicht aus für Anlagen, bei denen zur Sterilisierung ein Gemisch aus 12 % ETO und 88 % Freon R 12 verwendet wird. Dem beispielhaft dargestellten Molekularsieb 41' bzw. 42' mit kreisförmigem Querschnitt wird das zu trocknende Gasgemisch über einen oberen seitlichen Stutzen 43 zugeführt und über einen unteren seitlichen Stutzen 44 wird es abgezogen. Im Molekularsieb 41' bzw. 42' durchströmt das Gasgemisch den zwischen einem oberen und einem unteren Lochsieb 45, 46 befindlichen, mit einem geeigneten Adsorbens gefüllten Adsorptionsbereich 52, wo die im Gasgemisch enthaltene Feuchtigkeit adsorbiert wird. Bei Verwendung eines Gemisches, beispielsweise von ETO und Freon R 12 im Verhältnis von 12 : 88, verhindert die gute Kühlwirkung des Freon R 12 einen unzulässigen Anstieg der Temperatur im Molekularsieb 41' bzw. 42' . Um nun den bei Verwendung reinen ETO's zu beobachtenden gefährlichen Temperaturanstieg während der Feuchtigkeitsadsorption bis hin zur Explosionsgrenze des ETO zu verhindern, wird der Adsorptionsbereich 52 des Molekularsiebs 41 bzw. 42, wie in Fig. 3 und 4 dargestellt, in vier kühlbare, ringförmige Kammern unterteilt. Die Viererteilung und der ringförmige Aufbau sind jedoch nicht zwingend zu wählen. Innerhalb des äußeren Rings 47, an dem zur Regenerierung des Molekularsiebs 41 bzw. 42 ein Heizband 48 zur Erwärmung des beladenen Adsorbens zum Zwecke des Desorbierens angebracht sein kann, werden koaxial und konzentrisch zueinander ausgerichtete Rohre 49a bis 49d angeordnet. In diesem speziellen Ausführungsbeispiel handelt es sich dabei um vier Rohre 49a bis 49d, wobei der Innenbereich des inneren Rohrs 49a selbst keinen Adsorptionsbereich bildet. Wie in Fig. 3 dargestellt, ist jedes dieser Rohre 49a bis 49d von einer wendelförmigen, jeweils auf ein Rohr aufgeschweißten Kühlleitung 54 umgeben. Wie aus Fig. 1 ersichtlich, wurden die Kühlleitungen 54 und damit die Molekularsiebe 41, 42 vom Wasserbzw. Sole-Kreislauf der Kältemaschine 30 durchflossen. Im dargestellten Ausführungsbeispiel weisen die Rohre 49a bis 49d bei einem Durchmesser des Molekularsiebs von ca. 325 mm in aufsteigender Reihenfolge Durchmesser von 89, 140, 210 und 280 mm auf. In diesem Dimensionierungsbeispiel können beispielsweise aus dem äußeren ringförmigen Adsorptionsbereich, d.h. aus dem außerhalb des Rohrs 49d liegenden Bereich, etwa 34 % der insgesamt abzuführenden Wärmeleistung übernommen werden.

Um die Fortpflanzung von etwaigen vom Molekularsieb 41 bzw. 42 ausgehenden Zerfallsreaktionen des ETO's, zur Sterilisierkammer 10 zu verhindern, ist eine Flammsperre zwischen dem Trockner 40 und der Sterilisierkammer 10 vorgesehen. Mit einer Fortpflanzung solcher ETO-Zerfallsreaktion bis zum Tieftemperaturkondensator 50 ist wegen der dort herrschenden sehr tiefen Temperaturen nicht zu rechnen. Dabei wird der Vorkühler 20 durch entsprechende Dimensionierung der das ETO/Luft-Gemisch führenden Rohre als Flammsperre ausgebildet. Die Rohre sollten einen Strömungsquerschnitt mit höchstens 18 mm Durchmesser und bei Wahl eines solchen Durchmessers, eine Strömungslänge von mindestens 2 m aufweisen. Geringere Durchmesser bzw. größere Längen erhöhen natürlich die flammsperrende Wirkung.

**Patentansprüche**

1. Verfahren zur Rückgewinnung eines Sterilisiergases, insbesondere von Ethylenoxid, bei dem
   a) zu sterilisierende Materialien in einer Sterilisierkammer (10) mit dem Sterilisiergas sterilisiert werden,
   b) das aus der Sterilisierkammer (10) abgesaugte Sterilisiergas/Luft-Gemisch in einem Vorkühler (20) vorgetrocknet wird,
   c) das vorgetrocknete Sterilisiergas/Luft-Gemisch in einem Trockner (40) von Restfeuchtigkeit befreit wird, und
   d) das Sterilisiergas in einem Tieftemperaturkondensator (50) verflüssigt und bis zur Wiederverwendung in einem Lagerbehälter (80) gespeichert wird.
   **dadurch gekennzeichnet,**
   e) daß das Sterilisieren und die gesamte Rückgewinnung des Sterilisiergases bei Drücken unterhalb des die Anlage umgebenden Atmosphärendruckes abläuft.

2. Verfahren nach Anspruch 1, dadurch gekennzeichnet, daß in der Sterilisierkammer (10) ein Druck von höchstens 800 mbar herrscht.

3. Verfahren nach einem der Ansprüche 1 oder 2, dadurch gekennzeichnet, daß zur Befreiung des vorgetrockneten Sterilisiergas/Luft-Gemisches von Restfeuchtigkeit ein während der Adsorptionsphase gekühltes Molekularsieb (41, 42) eingesetzt wird.

4. Verfahren nach einem der Ansprüche 1 bis 3, dadurch gekennzeichnet, daß der Taupunkt des im Trockner (40) von Restfeuchtigkeit befreiten Sterilisergas/Luft-Gemisches zwischen -80° C und -100° C liegt.

5. Verfahren nach einem der Ansprüche 1 bis 4, dadurch gekennzeichnet, daß das dem Trockner (40; 41, 42) zugeführte Sterilisergas/Luft-Gemisch eine Temperatur zwischen 4° C und 10° C aufweist.

6. Verfahren nach einem der Ansprüche 1 bis 5, dadurch gekennzeichnet, daß das Sterilisiergas zu seiner Verflüssigung in dem mit flüssigem Stickstoff betriebenen Tieftemperaturkondensator (50) auf eine Temperatur zwischen -80° C und -130° C abgekühlt wird.

7. Verfahren nach einem der Ansprüche 1 bis 6, dadurch gekennzeichnet, daß die nicht kondensierbaren Bestandteile des Sterilisiergas/Luft-Gemisches durch eine Vakuumpumpe (75) aus der Anlage abgezogen werden.

8. Verfahren nach einem der Ansprüche 1 bis 7, dadurch gekennzeichnet, daß das im Tieftemperaturkondensator (50) verflüssigte Sterilisergas vom Tieftemperaturkondensator (50) direkt und/oder über einen nachgeschalteten Kondensatabscheider (60) in die Sterilisiergas-Vorlage (78) gelangt und von dort in den Lagerbehälter (80) abgepumt wird.

9. Anlage zur Rückgewinnung eines Sterilisiergases, insbesondere Ethylenoxid,
   a) mit einer Sterilisierkammer (10) zur Aufnahme der zu sterilisierenden Materialien,
   b) mit einem Vorkühler (20) zur Vortrocknung des Sterilisiergas/Luft-Gemisches aus der Sterilisierkammer (10),
   c) mit einem Trockner (40) zur Befreiung des Sterilisiergas/Luft-Gemisches von Restfeuchtigkeit,
   d) mit einem Tieftemperaturkondensator (50) zur Verflüssigung des Sterilisergases, und

   e) mit einem Lagerbehälter (80) zur Speicherung des verflüssigten Sterilisiergases,
   f) mit einer Rückführung (82) für das Sterilisgas aus dem Lagerbehälter (80) in die Sterilisierkammer (10),
   **gekennzeichnet**
   g) durch eine Vakuumpumpe (75) zur Aufrechterhaltung eines Unterdrucks in der Rückgewinnungsanlage.

10. Anlage nach Anspruch 9, dadurch gekennzeichnet, daß der Trockner (40) zumindest ein in seinem Adsorptionsbereich kühlbares Molekularsieb (41, 42) aufweist.

11. Anlage nach Anspruch 10, dadurch gekennzeichnet, daß das Molekularsieb (41, 42) eine kammerförmige Unterteilung aufweist, wobei zumindest jeweils eine Wand (49a-49d) einer solchen Kammer kühlbar ist.

12. Anlage nach Anspruch 11, dadurch gekennzeichnet, daß die Kammern des Molekularsiebes (41 bzw. 42) durch konzentrisch zueinander angeordnete Ringkammern gebildet werden, wobei zumindest jeweils eine Ringkammerwand wendelförmig angeordnete Kühlkanäle (54) aufweist.

13. Anlage nach Anspruch 12, dadurch gekennzeichnet, daß das Molekularsieb (41 bzw. 42) vier kühlbare Adsorptionskammern aufweist.

14. Analge nach einem der Ansprüche 9 bis 13, dadurch gekennzeichnet, daß der Trockner (40) zwei im Wechselbetrieb arbeitende Molekularsiebe (41 und 42) aufweist.

15. Anlage nach einem der Ansprüche 9 bis 14, dadurch gekennzeichnet, daß der zwischen der Sterilisierkammer (10) und dem Trockner (40) angeordnete Vorkühler (20) als Flammsperre für das Sterilisiergas/Luft-Gemisch ausgebildet ist.

16. Anlage nach Anspruch 15, dadurch gekennzeichnet, daß die vom Sterilisiergas/Luft-Gemisch durchströmten Rohre des Vorkühlers (20) durch die Abstimmung von Strömungslänge und Strömungsquerschnitt dieser Rohre als Flammsperren ausgebildet sind.

17. Anlage nach Anspruch 16, dadurch gekennzeichnet, daß die Rohre des Vorkühlers (20) ein Verhältnis von Strömungslänge zu Strömungsquerschnitt von mindestens 110 aufweisen.

Fig. 1

EP 0 516 963 A2

Fig. 3

Fig. 2

Fig. 4